# EUROPEAN PATENT SPECIFICATION

(11) **EP 2 750 682 B1**
(45) Date of publication and mention of the grant of the patent: **18.05.2016**
(21) Application number: 12772317.9
(22) Date of filing: 11.10.2012
(51) Int. Cl.: A61K 35/74, A61P 1/12, A61P 1/00, A61P 17/10, A61P 19/02, A61P 35/00

(54) **COMPOSITION COMPRISING ANAEROBICALLY CULTIVATED HUMAN INTESTINAL MICROBIOTA**
ZUSAMMENSETZUNG, ENTHALTEND ANAEROBISCH KULITIVIERTE MENSCHLICHE DARMFLORA
COMPOSITION COMPRENANT DE LA FLORE INTESTINALE CULTIVE DE FAÇON ANAEROBIQUE

(30) Priority: 11.10.2011 SE 1150940; 11.01.2012 SE 1250011
(43) Date of publication of application: 09.07.2014
(73) Proprietor: Achim Biotherapeutics AB, 162 44 Vällingby (SE)
(72) Inventor: BERSTAD, Arnold, N-0783 Oslo (NO); MIDTVEDT, Tore, N-1346 Gjettum (NO); NORIN, Elisabeth, S-162 44 Vällingby (SE); BENNO, Peter, S-181 33 Lidingö (SE); DAHLGREN, Atti-La, CH-2000 Neuchatel (CH)
(74) Representative: Fenix Legal KB
(86) International application number: PCT/EP2012/070177
(87) International publication number: WO 2013/053836

(56) References cited:
- WO-A1-02/07741
- WO-A1-2011/033310
- BAKKEN JOHAN S: "Fecal bacteriotherapy for recurrent Clostridium difficile infection", ANAEROBE,, vol. 15, no. 6, 1 January 2009 (2009-01-01), pages 285-289, XP009161367, ISSN: 1095-8274
- JOHANNES AAS ET AL: "Recurrent Clostridium difficile Colitis: Case Series Involving 18 Patients Treated with Donor Stool Administered via a Nasogastric Tube", CLINICAL INFECTIOUS DISEASES, vol. 36, no. 5, 1 March 2003 (2003-03-01), pages 580-585, XP55043539, ISSN: 1058-4838, DOI: 10.1086/367657
- BORODY T J ET AL: "Treatment of ulcerative colitis using fecal bacteriotherapy", JOURNAL OF CLINICAL GASTROENTEROLOGY, RAVEN PRESS LTD., NEW YORK, NY, US, vol. 37, no. 1, 1 January 2003 (2003-01-01), pages 42-47, XP008093566, ISSN: 0192-0790, DOI: 10.1097/00004836-200307000-00012
- Sebastian Amaral Anders: "Mit Fäkalien heilen", Frankfurter Rundschau , 2 May 2011 (2011-05-02), 2 May 2011 (2011-05-02), XP002686975, Retrieved from the Internet: URL:http://www.fr-online.de/wissenschaft/b akterientherapie-mit-faekalien-heilen,1472 788,8400888.html [retrieved on 2012-11-08]
- SERINO M ET AL: "Intestinal microflora and metabolic diseases", DIABETES & METABOLISM, PARIS, AMSTERDAM, NL, vol. 35, no. 4, 1 September 2009 (2009-09-01), pages 262-272, XP026642909, ISSN: 1262-3636, DOI: 10.1016/J.DIABET.2009.03.003 [retrieved on 2009-05-05]

## Description

### FIELD OF THE INVENTION

The present invention relates to an anaerobic micro-ecological system comprising anaerobically cultivated human intestinal microbiota. The present invention also relates to a composition of anaerobically cultivated microbiota that constitutes a functional seeding culture for re-establishing normality of a disturbed human microbiome and gastrointestinal functions.

The present invention further relates to the composition for use as a pharmaceutical and for use as prophylaxis of diseases and a method for preparing the composition.

### TECHNICAL BACKGROUND

The gastrointestinal microbiota consists of several complex micro-ecological systems, where interactions in-between the individual microbial strains and between the microbiota and the host takes place. The interactions with the host occur through direct contact with the mucosal wall and indirectly e.g. through metabolites and signalling substances.

This intestinal ecological balance can be disturbed by external and internal physical, chemical and biological factors or agents, the consequence of which can be local e.g. disturbed metabolism, gastrointestinal motility alteration, altered functions/crosstalks, and/or lead to systemic effects involving other organ systems.

Clostridium difficile associated diarrhoea CDAD was first described around 1/3 of a century ago. However, in spite of thousands of publications in Pub Med of the pathogenesis, epidemiology, clinical diagnosis and various therapeutic approaches CDAD continues to persist as a costly, leading cause of infectious health-care-associated gastrointestinal illness and the problems are assumed to increase worldwide. It was recently stated that today we experienced a tenfold increase of CDAD in Europe, USA and Canada. Increasing therapeutic failures of metronidazole and vancomycin treatments are reported and new antibiotics, as fidaxomicin, oritavancin, nitazoxanide, REP3123, and NVB303 are on their way to the market. However, it has to be recognized that even before these drugs have been used, a certain number of Clostridium difficile strains are expressing a reduced sensitivity to these new drugs. Additionally, whatever their clinical efficacy may turn out to be, they will certainly be expensive in use.

It has been stated that some patients continue to manifest relapsing diarrhoea after completed treatment with novel drugs, and therefore other strategies have been focused on e.g. faecal transplantation from a close relative or a synthetic composition comprising a mixture of different bacteria.

WO 02/07741 describes a synthetic composition comprising a preparation of a predetermined flora for the treatment of gastrointestinal disorders.
WO 2011/033310 describes encapsulated dosage form comprising flora extracted from faeces for use in the treatment of gastrointestinal disorders.

Faecal transplantation is a known method, but not without risk. Until now faecal material from donors of close relatives has been used. A main problem with this procedure is the risk of transmission of genes resistant to certain antibiotics. The transfer of such genes may have serious consequences for the patient when antibiotic treatment is needed in the future. Another main problem with this therapy is the risk of transmission of potentially contagious agents present in the donor's faeces, which argue for careful screening of the faeces to be given. This screening is time-consuming and expensive. Further this type of treatment is perceived by many as highly unesthetical.

Previous publications mentioning faecal transplantation:
- Johan S. Bakken Fecal bacteriotherapy for recurrent Clostridium difficile infection Anaerobe 15 (2009) 285-289.
- C Jorup-Rönström, A Håkansson, A-K Person, T Midtvedt, E Norin. Feceskultur framgångsrik terapi vid Clostridium difficile-diarré. Läkartidningen nr 46 2006 volym 103, 3603-3605.
- A. Gustafsson, A. Berstad, S. Lund-Tønnesen, T. Midtvedt, E. Norin. The Effect of Faecal Enema on Five Microflora-Associated Characteristics in Patients with Antibiotic-Associated Diarrhoea. Scand J Gastroenterol 1999;34:580-586.
- A. Gustavsson, S. Lund-Tonnesen, A. Berstad, T. Midtvedt & E. Norin Faecal Short-Chain Fatty Acids in Patients with Antibiotic-Associated Diarrhoea, before and after Faecal Enema Treatment. Scand J Gastroenterol 1998;33:721-727.

### SUMMARY OF THE INVENTION

The present invention relates to a composition comprising strictly anaerobically cultivated human intestinal microbiota selected from at least three of the fow phyla Bacterioidete, Firmicutes, Proteobacteria and Actinobacteria for use in the prevention and treatment of diseases. The present invention further relates to a method to prepare such a composition. Also described is the administration of a therapeutic or prophylactic amount of the invention to a human.

Also described is a composition comprising anaerobically cultivated intestinal microbiota that constitutes a functional seeding culture for re-establishing normality of a disturbed human microbiota and gastrointestinal functions and the use of such composition for the prevention and treatment of disease.

This invention also relates to a composition according to the claims for use in the pharmaceutical treatment of gastrointestinal and/or systemic/metabolic disorders that has its origin in a disturbed or dysfunctional gastrointestinal ecosystem.

The invention also relates to the use of such an anaerobically cultivated human microbiota for pharmaceutical treatment or prevention of disease. The composition may also contain other microbial factors (e.g. bacteriophages) or signal substances active against diseases.

### DETAILED DESCRIPTION OF THE INVENTION

The present invention is defined by the claims.

A first aspect of the present invention is a composition comprising anaerobically cultivated human intestinal microbiota as defined in the claims.

In the present application the term "microbiota" is used for the microbial flora, i.e. the microorganisms that typically inhabit the gastrointestinal system.

The composition according to the present invention constitutes a functional cultivate for re-establishing normality of a disturbed human microbiota and gastrointestinal functions. This composition has surprisingly shown to be useful as in the treatment or prophylaxis of disease.

The composition according to the present invention interacts in a synergistic way with the damaged intestinal microbiota and re-establish its physiological and metabolic functions. The invention may also be used as a prophylactic to avoid the disturbance of the intestinal microbiota in the first place.

The composition contains anaerobic bacteria of at least three of the following four Phyla: Bacterioidetes, Firmicutes, Proteobacteria and Actinobacteria.

According to another embodiment the composition is free from the following microbes: hepatitis viruses A, B and C, cytomegalo virus, Epstein-Barr virus, human immunodeficiency virus (HIV), Calici- and Rotavirus, Salmonella, Shigella, Campylobacter, Yersinia, and protozoan cysts. Preferably it is also free from material selected from Extended spectrum- and Metallo-beta-lactamases, metabolites of pharmaceutical substances, and xenobiotics.

Preferably the microbiota in the composition according to the present invention have not been exposed to antibiotics taken into clinical use after 1995.

This invention comprises an anaerobically cultivated human intestinal microbiota for the pharmaceutical treatment of gastrointestinal and/or systemic/metabolic disorders that has its origin in a dysfuntional gastrointestinal ecosystem.

An advantage with the composition according to the present invention is that it consists of functional micro-ecological system that is stable and homogenous during 15 years of cultivation. No other bacterial strains enter the system during cultivation. The microbial strains in the micro-ecological system interact and communicate with each other. A micro-ecological system is homogenous.

The functional ecologic system according to the present invention has the ability to re-establish and/or stabilise intestinal microbiota and/or normal intestinal functions.

Another aspect of the present invention is a composition comprising a micro-ecological system comprising anaerobically cultivated human intestinal microbiota for use in the treatment or prophylaxis of diseases.

Preferably, the bacterial content of the composition is more than 10⁶ per ml medium, more preferably more than 10⁹ per ml medium.

In one embodiment of the present invention the composition comprises an anaerobic micro-ecological system obtained from faeces from a single donor. Cultivation of faeces from a healthy single donor is necessary for obtaining a functional micro-ecological system that is stable over time.

Preferably, the donor is a healthy individual with a well-functional gastrointestinal system.

Preferably, the faeces is obtained from a single stool sample. Also preferably, the faeces were obtained prior to 1995.

In a preferred embodiment of the present invention, the composition is kept under strict anaerobic conditions until the final administration. Also, preferably the composition is kept under anaerobic conditions during the final administration.

Another aspect of the present invention is a method for preparing a composition comprising an anaerobic micro-ecological system comprising anaerobically cultivated human intestinal microbiota according to the claims.

The composition according to the present invention has been cultivated under strict anaerobic conditions. By anaerobic cultivation the proliferation of the anaerobic bacteria is promoted, and further proliferation of aerobic bacterial is suppressed.

In a preferred embodiment of the present invention, the composition is prepared by cultivating a stool sample obtained from a healthy individual. Preferably, the healthy individual has normal functional values of coprostanol, urobilins, mucin, fecal tryptic activity, short chain fatty acids and beta-aspartylglycine. Also preferably, the sample should be obtained prior to 1995. More preferably the stool sample is investigated to be free from hepatitis viruses A, B and C, cytomegalovirus, Epstein-Barr virus, human immunodeficiency virus (HIV), Calici- and Rotavirus. Furthermore, the stool sample is screened to be free from Salmonella, Shigella, Campylobacter, Yersinia, Clostridium difficile and the presence of protozoan cysts.

The cultivating medium used in the method according to the invention promotes the proliferation of the anaerobic bacteria of at least three of the following four Phyla: Bacterioidetes, Firmicutes, Proteobacteria and Actinobacteria.

In one embodiment the cultivation medium is a yeast based medium, preferably a bacteriological peptone yeast based medium. The cultivation medium comprises added cholesterol, preferably cholesterol of animal origin. Even more preferably the medium comprises added freeze dried hen yolk, preferably in an amount of from 0.5 to 5 % more preferably 1.25 % w/v (weight per volume).

Preferably, the cultivation contains an anaerobic indicator, preferably resazurin.

The cultivating medium used in the method according to the invention promotes the proliferation of the anaerobic bacteria of at least three of the following four Phyla: Bacterioidetes, Firmicutes, Proteobacteria and Actinobacteria in one and the same sample. Further, the preferred cultivating conditions suppresses the proliferation of aerobic bacteria.

In a preferred embodiment the composition is re-cultivated under strict anaerobic conditions, preferably under nitrogen flow every second week.

Preferably, the faecal sample is anaerobically cultivated for at least 15 years.

Cultivating a faecal sample over time according to the method of the present invention promotes the proliferation of the desired bacteria, while aerobic bacteria are suppressed.

An advantage with the method according to the present invention is that it enables proliferation of all desired bacteria in one and the same sample. Thus, no mixing of different cultures is necessary. This has the advantage that the obtained composition comprises a micro-ecological system that has a stabilizing effect on the intestinal microbiota and is able to re-establish the communication between the host and the intestine. Further, by anaerobically cultivating microbiota in one sample, according to the claimed invention, the same micro-ecological system is reproduced in each composition with retained physiological and/or pharmaceutical effect over a long time, such as over at least 15 years.

Further, the micro-ecological system according to the present invention is cultivated under strict anaerobic conditions.

The human intestinal flora consists of several hundreds of different microbes, of which the vast majority are strictly anaerobic.
In one embodiment of the invention the following bacterial species together comprise more than 1% preferably more than 20%, and more preferably more than 50% of the total bacterial content of the composition;
Bacteria;"Actinobacteria";Actinobacteria;Coriobacteridae;Coriobacteriales;"Corioba cterineae";Coriobacteriaceae;Collinsella,
Bacteria;"Actinobacteria";Actinobacteria;Coriobacteridae;Coriobacteriales;"Corioba cterineae";Coriobacteriaceae;Eggerthella,
Bacteria;"Actinobacteria";Actinobacteria;Coriobacteridae;Coriobacteriales;"Corioba cterineae";Coriobacteriaceae;Olsenella,
Bacteria;"Bacteroidetes";"Bacteroidia";"Bacteroidales";"Porphyromonadaceae";Par abacteroides,
Bacteria;"Bacteroidetes";"Bacteroidia";"Bacteroidales";"Prevotellaceae";Prevotella, Bacteria;"'Bacteroidetes";"Bacteroidia"-,"Bacteroidales";"Rikenellaceae";Alistipes, Bacteria;"Bacteroidetes";"Bacteroidia";"Bacteroidales";Bacteroidaceae;Bacteroides ,
Bacteria;"Firmicutes";"Bacilli";"Lactobacillales";"Enterococcaceae';Enterococcus, Bacteria;"Firmicutes";"Clostridia";Clostridiales;"Eubacteriaceae";Eubacterium Bacteria.;"Firmicutes";"Clostridia";Clostridiales;"Eubacteriaceae";unclassified_"Euba cteriaceae",
Bacteria;"Firmicutes";"Clostridia";Clostridiales;"Lachnospiraceae";Anaerostipes, Bacteria;"Firmicutes";"Clostridia";Clostridiales;"Lachnospiraceae";Dorea, Bacteria;"Firmicutes";"Clostridia";Clostridiales;,"Lachnospiraceae",Roseburia, Bacteria;"Firmicutes";"Clostridia";Clostridiales;"Lachnospiraceae";unclassified_"Lac hnospiraceae",
Bacteria;"Firmicutes";"Clostridia";Clostridiales;"Peptostreptococcaceae";Sporacetig enium,
Bacteria;"Firmicutes";"CIostridia";Clostridiales;"Peptostreptococcaceae" ;unclassifie d_"Peptostreptococcaceae", Bacteria;"Firmicutes';"Clostridia";Clostridiales;"Ruminococcaceae";Anaerofilum, Bacteria;"Firmicutes";"Clostridia";Clostridiales;"Ruminococcaceae";Anaerotruncus, Bacteria;"Firmicutes";"Clostridia";Clostridiales;"Ruminococcaceae";Oscillibacter, Bacteria;"Firmicutes";"Clostridia";Clostridiales;"Ruminococcaceae";unclassified_"R uminococcaceae",
Bacteria;"Firmicutes";"Clostridia";Clostridiales;Clostridiaceae;"Clostridiaceae1";Clo stridium,
Bacteria;"Firmicutes";"Clostridia,,;Clostridiales;Clostridiaceae;"Clostridiaceae1";Sar cina,
Bacteria;"Firmicutes";"Clostridia";Clostridiales;Clostridiaceae;"Clostridiaceae1";uncl assified_"Clostridiaceae1",
Racteria;"Firmicutes";"Clostridia";Clostridiales;lncertaeSedisXI;Anaerococcus, Bacteria;"Firmicutes";"Clostridia";Clostridiales;IncertaeSedisXI;Finegoldia, Bacteria;"Firmicutes";"Clostridia";Clostridiates;IncertaeSedisXI;Peptoniphilus, Bacteria;"Firmicutes";"Clostridia";Clostridiales;IncertaeSedisXI;unclassified_Incerta eSedisXI,
Bacteria;"Firmicutes";"Clostridia";Clostridiales;IncertaeSedisXIII;Anaerovorax, Bacteria;"Firmicutes";"Clostridia";Clostridiales;IncertaeSedisXIII;Mogibacterium, Bacteria;"Firmicutes";"Clostridia";Clostridiales;IncertaeSedisXIV;Blautia, Bacteria;"Firmicutes";"Clostridia";Clostridiales;unclassifed_Clostridiales, Bacteria;"Firmicutes";"Clostridia";Clostridiales;Veillonellaceae;Veillonella, Bacteria;"Firmicutes";"Erysipelotrichi";"Erysipelotrichales";Erysipelotrichaceae;Hold emania,
Bacteria;"Proteobacteria";Betaproteobacteria;Burkholderiales;Alcaligenaceae;Para sutterella,

Diseases that can be treated or prevented by the invention are for example diarrhoeas following antibiotic treatment, especially diarrhoeas caused by Clostridium difficile which can be very long lasting and difficult to treat by conventional methods and can in rare cases lead to death. Other therapeutic areas are Irritable Bowel Syndrome (IBS), Celiac Disease and Inflammatory Bowel Diseases for example Ulcerative Colitis, Crohn's Disease, Microscopic Colitis, and Pauchitis. Another treatment area is iatrogenic disturbance/dysbiosis of the intestinal microbiome, e.g., following radiation therapy, chemotherapy and in connection with transplantations. Examples of other diseases that can be treated or prevented with the invention are: neurological diseases for example Parkinson's Disease, Alzheimer's Disease, Lou Gehrig's Disease ALS-Amyotrophic Lateral Sclerosis, Multiple Sclerosis; behavioural/psychiatric disorders e.g. Autism, Asperger's Syndrome , Attention Deficit Hyperactivity Disorder (ADHD) and Depression; Rheumatologic diseases e.g. Rheumatoid arthritis; and systemic/metabolic disorders for example: Hypertension, Obesitas and type-2 Diabetes, and neoplastic/tumour diseases of the gastrointestinal tract. The invention can also be used in the treatment of Chronic Fatigue Syndrome. The invention can also be used in the treatment of dermatological diseases such as acne vulgaris.

Also described is the use of such a anaerobically cultivated human microbiota for the production of a pharmaceutical treatment or prevention of disease.

The composition according to the present invention maybe administrated to a human by a device selected from a naso-duodenal tube, gastroscope, colo/sigmoideoscope, and enema, or in freeze dried form in a suitable galenic preparation, e.g. gastric acid resistant capsule, nano-encapsulation or suppository.

In one embodiment the composition may also contain other microbial factors (e.g. bacteriophages) or signalling substances active against diseases.

### Forms of preparations

### The composition may be provided in the following forms of preparations:

1. Freshly thawn from frozen solution
2. Freeze-dried cultivated product
3. Fresh from the culture
4. Live micro-encapsulated culture

### Dosage

The invention is applied to the human gastro-intestinal tract at least one time per day during the course of a number of days, e.g. 1-30 days, depending on the nature, cause and severity of the problem. Per administration 1-100 ml of the product is given, preferably 20-40 ml, or the equivalent amount in freeze-dried form.

### Means of administration

The product can preferably be administered according to the following non limiting examples: in the form of a solution through e.g. a naso-duodenal tube, gastroscope, colo/sigmoideoscope, enema or in freeze dried form in a suitable galenic preparation, e.g. a gastric acid resistant capsule, nano-encapsulated or a suppository.

### Other ingredients (vehicles)

Other substances and components which facilitates the administration or supports the effect of the invention may be added to the composition.
The treatment with the invention may or may not be preceded by an antibiotic treatment and/or motility reducing substances.

The invention is described by the following non limiting examples.

### Example1

Microbiota according to the present invention were obtained from a stool sample from a healthy single donor prior to 1995. Both the donor and the stool were thoroughly examined. The functional status of the donor's intestinal microbiota were found to have normal functional values of coprostanol, urobilins, mucin, fecal tryptic activity, short chain fatty acids and beta-aspartylglycine. The tests for hepatitis viruses A, B and C, cytomegalovirus, Epstein-Barr virus, human immunodeficiency virus (HIV), Calici- and Rotavirus were all negative. Furthermore, the faeces was screened for presence of Salmonella, Shigella, Campylobacter, Yersinia, Clostridium difficile and protozoan cysts, and all these investigations were negative.

A sample of faeces from the interior of the stool sample (Bristol scale type 2) was taken from the above-mentioned healthy donor and immediately suspended in 30 ml of a pre-reduced sterilized bacteriological peptone yeast based medium (Difco, USA), with addition of 1.25 % (w/v) freeze dried hen yolk, Fresenius-Kabi Sweden with resazurin as an anaerobic indicator. This composition was re-cultivated under nitrogen flow every second week. The bacterial content of the cultivated product were found to be > 10⁹ per ml medium.

The product contained the following species:
Bacteria;"Actinobacteria";Actinobacteria;Coriobacteridae;Coriobacteriales;"Corioba cterineae";Coriobacteriaceae;Collinsella,
Bacteria;"Actinobacteria";Actinobacteria;Coriobacteridae;Coriobacteriales;"Corioba cterineae";Coriobacteriaceae;Eggerthella,
Bacteria;"Actinobacteria";Actinobacteria;Coriobacteridae;Coriobacteriales;"Corioba cterineae";Coriobacteriaceae;Olsenella,
Bactecia;"Bacteroidetes";"Bacteroidia";"Bacteroidales";"Porphyromonadaceae";Par abacteroides,
Bacteria;"Bacteroidetes";"Bacteroidia" ;"Bacteroidales";"Prevotellaceae";Prevotella,
Bacteria;"Bacteroidetes";"Bacteroidia";"Bacteroidales";"Rikenellaceae";Alistipes,
Bacteria;"Bacteroidetes";"Bacteroidia";"Bacteroidales";Bacteroidaceae;Bacteroides
Bacteria;"Firmicutes";"Bacilli";"Lactobacillales";"Enterococcaceae";Enterococcus,
Bacteria;"Firmicutes";"Clostridia";Clostridiales;"Eubacteriaceae";Eubacterium
Bacteria;"Firmicutes";"Clostridia';Clostridiales;"Eubacteriaceae";unclassified_"Euba cteriaceae",
Bacteria;"Firmicutes";"Clostridia";Clostridiales;"Lachnospifaceae";Anaerostipes,
Bacteria;"Firmicutes";"Clostridia";Clostridiales;"Lachnospiraceae";Dorea,
Bacteria;"Firmicutes";"Clostridia";Clostridiates;"Lachnospiraceae";Roseburia,
Bacteria;"Firmicutes";"Clostridia";Clostridiales;"Lachnospiraceae";unclassified_"Lac hnospiraceae",
Bacteria;"Firmicutes";"Clostridia";Clostridiales;"Peptostreptococcaceae";Sporacetig enium,
Bacteria;"Firmicutes";"Clostridia";Clostridiales;"Peptostreptococcaceae";unclassifie d_"Peptostreptococcaceae",
Bacteria;"Firmicutes";"Clostridia";Clostridiales;"Ruminococcac,eae";Anaerofilum,
Bacteria;"Ficmicutes";"Clostridia";Clostridiales;"Ruminococcaceae";Anaerotruncus,
Bacteria;"Firmicutes";"Clostridia";Clostridiales;"Ruminococcaceae";Oscillibacter,
Bacteria;"Firmicutes";"Clostridia";Clostridiales;"Ruminococcaceae";unclassified-"R uminococcaceae",
Bacteria;"Firmicutes";"Clostridia";Clostridiales;Clostridiaceae;"Clostridiaceae1 ";Clo stridium,
Bacteria;"Firmicutes";"Clostridia";Clostridiales;Clostridiaceae;"Clostridiaceae1 ";Sar cina,
Bacteria;"Firmicutes";"Clostridia";Clostridiales;Clostridiaceae;"Closstridiaceae1";uncl assified_"Clostridiaceae1",
Bacteria;"Firmicutes";"Clostridia";Clostridiales;lncertaeSedisXI;Anaerococcus,
Bacteria;"Firmicutes";"Clostridia";Clostridiales;IncertaeSedisXI;Finegoldia,
Bacteria;"Firmicutes";"Clostridia";Clostridiales;IncertaeSedisXI;Peptoniphilus,
Bacteria;"Firmicutes";"Clostridia";Clostridiales;IncertaeSedisXI;unclassified_Incerta eSedisXI,
Bacteria;"Firmicutes";"Clostridia";Clostridiales;IncertaeSedisXIII;Anaerovorax,
Bacteria;"Firmicutes";"Clostridia";Clostridiales;lncertaeSedisXIII;Mogibacterium,
Bactecia;"Firmicutes";"Clostridia";Clostridiales;IncertaeSedisXIV;Blautia,
Bacteria;"Firmicutes";"Clostridia";Clostridiales;unclassified_Clostridiales,
Bacteria;"Firmicutes";"Clostridia";Clostridiafes;Veillonellaceae;Veillonella,
Bacteria;"Firmicutes";"Erysipelotrichi";"Erysipelotrichales";Erysipelotrichaceae;Hold emania, and
Bacteria;"Proteobacteria";Betaproteobacteria;Burkholderiales;Alcaligenaceae;Para sutterella,

After addition of 10% glycerol the composition can be stored at -70°C.

### Example 2

The fresh or frozen stool sample from an individual who has not been exposed to antibiotics or antibiotic resistance genes e.g. through a bacterial infection, is inoculated under anaerobic conditions on to 30 ml peptone-yeast medium (Difco,USA), containing cholesterol of animal origin ( e.g. 1.25% freeze-dried hen yolk, Fresenius-Kabi,Sweden), with resazurin as an anaerobic indicator. This composition is re-cultivated every second week. The bacterial content of the cultivated product should be > 10⁶ preferably > 10⁹ per ml medium. After addition of 10% glycerol the composition can be stored at -70°C.

### Example 3

A number of 32 patients, aged 27-94 years (20 females, median age 78 years and 12 males, median age 75 years) were treated with a composition according to the invention. Out of these patients, 22 had underlying chronic diseases as cancer (breast, colon, prostate) and cardiovascular or pulmonary diseases. In 11 patients cephalosporins were given, in 9 patients clindamycin were given, in 4 patients ciprofloxacin were given, in 2 patients isoxazolylpenicillin were given, another 3 patients were given some other antibiotics, and finally, 1 patient was given azitromycin when recurrent Clostridium Difficile infection (RCDI) first was diagnosed. 2 patients did not obtain specific antibiotic pretreatment before receiving the composition according to the invention.

Routinely, a volume of 30 ml of the invention according to Example 1 was given rectally by a rectally introduced catheter in Sigmoideum or by colonoscopy. Analysis of clostridium toxin was performed according to general practice at Karolinska Hospital, Huddinge, Sweden. Out of the 32 patients receiving the invention, 22 patients were cured. These 22 patients experienced a dramatic improvement in quality of life from living with diarrhoea during several months to a life with normal bowel habits within some few days. Fifteen of these patients received the invention only once, the remaining received the invention one to two more times. Four more patients experienced a considerable improvement, and in the remaining 4 patients repeated antibiotic therapies had to be given. Another 2 patients with unknown cause of the diarrhoea were also cured. The follow-up time was been 5-68 months, median 26 months. No adverse effects by the procedure were observed.

### Example 4

Ten patients with post-infectious gastroenteritis related to Giardia lamblia, were suffering from IBS like problems. Conventional IBS treatment was not successful. It was decided that these patients should receive the composition according to the invention. One dose of 30 ml was administrated over three consecutive days, using a gastro-jejunal tube. Surprisingly, all patients recovered shortly after the administration of the invention, and remained without symptoms for a long period of time.

## Claims

1. A composition comprising strictly anaerobically cultivated human intestinal microbiota selected from at least three of the four Phyla,Bacterioidetes, Firmicutes, Proteobacteria and Actinobacteria for use in the prevention and treatment of diseases.

2. The composition according to claim 1, **characterised in that** it is free from extended spectrum-and metallo-beta-lactamases, metabolites of pharmaceutical substances, or xenobiotics.

3. The composition according to any one of claims 1-2, **characterised in that** it is free from the following microbes: hepatitis viruses A, B and C, cytomegalo virus, Epstein-Barr virus, human immunodeficiency virus (HIV), Calici- and Rotavirus, Salmonella, Shigella, Campylobacter, Yersinia, and protozoan cysts.

4. The composition according to any one of claims 1-3, **characterised in that** the following bacterial species together comprise more than 1 % of the total bacterial content of the invention:
Bacteria;"Actinobacteria";Actinobacteria;Coriobacteridae;Coriobacteriales;"Co riobacterineae";Coriobacteriaceae;Collinsella,
Bacteria;"Actinobacteria";Actinobacteria;Coriobacteridae;Coriobacteriales;"Co riobacterineae";Coriobacteriaceae; Eggerthella,
Bacteria;"Actinobacteria";Actinobacteria;Coriobacteridae;Coriobacteriales;"Co riobacterineae";Coriobacteriaceae;Olsenella,
Bacteria;"Bacteroidetes";"Bacteroidia";"Bacteroidales";"Porphyromonadaceae ";Parabacteroides,
Bacteria;"Bacteroidetes";"Bacteroidia";"Bacteroidales";"Prevotellaceae"; Prevotella,
Bacteria;"Bacteroidetes";"Bacteroidia";"Bacteroidales";"Rikenellaceae";Alistipes,
Bacteria;"Bacteroidetes";"Bacteroidia";"Bacteroidales";Bacteroidaceae; Bacteroides,
Bacteria;"Firmicutes";"Bacilli";"Lactobacillales";"Enterococcaceae"; Enterococcus,
Bacteria;"Firmicutes";"Clostridia";Clostridiales;"Eubacteriaceae";Eubacterium,
Bacteria;"Firmicutes";"Clostridia";Clostridiales;"Eubacteriaceae";unclassified_ "Eubacteriaceae",
Bacteria;"Firmicutes";"Clostridia";Clostridiales;"Lachnospiraceae";Anaerostipes,
Bacteria;"Firmicutes";"Clostridia";Clostridiales;"Lachnospiraceae";Dorea,
Bacteria;"Firmicutes";"Clostridia";Clostridiales;"Lachnospiraceae";Roseburia,
Bacteria;"Firmicutes";"Clostridia";Clostridiales;"Lachnospiraceae";unclassified _"Lachnospiraceae",
Bacteria;"Firmicutes";"Clostridia";Clostridiales;"Peptostreptococcaceae";Spor acetigenium,
Bacteria;"Firmicutes";"Clostridia";Clostridiales;"Peptostreptococcaceae";uncla ssified_"Peptostreptococcaceae",
Bacteria;"Firmicutes";"Clostridia";Clostridiales;"Ruminococcaceae";Anaerofilum,
Bacteria;"Firmicutes";"Clostridia";Clostridiales;"Ruminococcaceae"; Anaerotruncus,
Bacteria;"Firmicutes";"Clostridia";Clostridiales;"Ruminococcaceae";Oscillibacter,
Bacteria;"Firmicutes";"Clostridia";Clostridiales;"Ruminococcaceae";unclassified_"Ru minococcaceae",
Bacteria;"Firmicutes";"Clostridia";Clostridiales;Clostridiaceae;"Clostridiaceae1 ";Clostridium,
Bacteria;"Firmicutes";"Clostridia";Clostridiales;Clostridiaceae;"Clostridiaceae1 ";Sarcina,
Bacteria;"Firmicutes";"Clostridia";Clostridiales;Clostridiaceae;"Clostridiaceae1 ";unclassified_"Clostridiaceae1",
Bacteria;"Firmicutes";"Clostridia";Clostridiales;IncertaeSedisXI;Anaerococcus,
Bacteria;"Firmicutes";"Clostridia";Clostridiales;IncertaeSedisXI;Finegoldia,
Bacteria;"Firmicutes";"Clostridia";Clostridiales;IncertaeSedisXI;Peptoniphilus,
Bacteria;"Firmicutes";"Clostridia";Clostridiales;IncertaeSedisXI;unclassified_In certaeSedisXI,
Bacteria;"Firmicutes";"Clostridia";Clostridiales;IncertaeSedisXIII;Anaerovorax,
Bacteria;"Firmicutes";"Clostridia";Clostridiales;IncertaeSedisXIII;Mogibacterium,
Bacteria;"Firmicutes";"Clostridia";Clostridiales;IncertaeSedisXIV;Blautia,
Bacteria;"Firmicutes";"Clostridia";Clostridiales;unclassified_Clostridiales,
Bacteria;"Firmicutes";"Clostridia";Clostridiales;Veillonellaceae;Veillonella,
Bacteria;"Firm icutes";"Erysipelotrichi";"Erysipelotrichales";Erysipelotrichaceae ;Holdemania, and
Bacteria;"Proteobacteria";Betaproteobacteria;Burkholderiales;Alcaligenaceae; Parasutterella.

5. The composition according to any one of claims 1-3, **characterised in that** the bacterial content of the composition comprises one or more of the bacterial groups mentioned in claim 4.

6. The composition according to any one of claims1-5 for use in the treatment of diarrhoea, such as Irritable Bowel Syndrome (IBS), or antibiotic associated diarrhea(AAD), especially related to Clostridium difficile , Clostridium difficile associated diarrhoea ( CDAD) and recurrent Clostridium difficile infections (RCDI).

7. The composition according to any one of claims 1-6 for use in administration to a human by a device selected from a naso-duodenal tube, gastroscope, colo/sigmoideoscope, and enema, or in freeze dried form in a suitable galenic preparation, e.g. gastric acid resistant capsule, nano-encapsulated or suppository.

8. A method for preparing a composition comprising strictly anaerobically cultivated human intestinal microbiota selected from at least three of the four Phyla, Bacterioidetes, Firmicutes, Proteobacteria and Actinobacteria, the method **characterised by** anaerobic cultivation of a stool sample in a cultivation medium that promotes the proliferation of the anaerobic bacteria of at least three of the following four Phyla: Bacterioidetes, Firmicutes, Proteobacteria and Actinobacteria, and **characterised in that** aerobic bacteria are suppressed during cultivation.

## Patentansprüche

1. Zusammensetzung, welche streng anaerob kultivierte humane intestinale Mikrobiota, ausgewählt aus wenigstens drei der vier Stämme (Phyla) Bacterioidetes, Firmicutes, Proteobacteria und Actinobacteria, umfasst, zur Verwendung bei der Vorbeugung und Behandlung von Krankheiten.

2. Zusammensetzung gemäß Anspruch 1, **dadurch gekennzeichnet, dass** sie frei von Extended-Spectrum- und Metallo-beta-Laktamasen, Metaboliten pharmazeutischer Substanzen oder Xenobiotika ist.

3. Zusammensetzung gemäß irgendeinem der Ansprüche 1 bis 2, **dadurch gekennzeichnet, dass** sie frei von folgenden Mikroben ist:
Hepatitis-Viren A, B und C, Zytomegalie-Virus, Epstein-Barr-Virus, humanes Immundefizienz-Virus (HIV), Calici- und Rotavirus, Salmonella, Shigella, Campylobacter, Yersinia und Protozoen-Zysten.

4. Zusammensetzung gemäß irgendeinem der Ansprüche 1 bis 3, **dadurch gekennzeichnet, dass** die folgenden bakteriellen Spezies zusammen mehr als 1 % des gesamten Bakteriengehalts der Erfindung ausmachen:
Bacteria; "Actinobacteria"; Actinobacteria; Coriobacteridae; Coriobacteriales; "Coriobacterineae"; Coriobacteriaceae; Collinsella,
Bacteria; "Actinobacteria"; Actinobacteria; Coriobacteridae; Coriobacteriales; "Coriobacterineae"; Coriobacteriaceae; Eggerthella,
Bacteria; "Actinobacteria"; Actinobacteria; Coriobacteridae; Coriobacteriales; "Coriobacterineae"; Coriobacteriaceae; Olsenella,
Bacteria; "Bacteroidetes"; "Bacteroidia"; "Bacteroidales"; "Porphyromonadaceae"; Parabacteroides,
Bacteria; "Bacteroidetes"; "Bacteroidia"; "Bacteroidales"; "Prevotellaceae"; Prevotella,
Bacteria; "Bacteroidetes"; "Bacteroidia";"Bacteroidales"; "Rikenellaceae"; Alistipes,
Bacteria; "Bacteroidetes"; "Bacteroidia"; "Bacteroidales"; Bacteroidaceae;
Bacteroides,
Bacteria; "Firmicutes"; "Bacilli"; "Lactobacillales"; "Enterococcaceae";
Enterococcus,
Bacteria; "Firmicutes"; "Clostridia"; Clostridiales; "Eubacteriaceae"; Eubacterium,
Bacteria; "Firmicutes"; "Clostridia"; Clostridiales; "Eubacteriaceae"; nicht klassifizierte "Eubacteriaceae",
Bacteria; "Firmicutes"; "Clostridia"; Clostridiales; "Lachnospiraceae"; Anaerostipes,
Bacteria; "Firmicutes"; "Clostridia"; Clostridiales; "Lachnospiraceae"; Dorea,
Bacteria; "Firmicutes"; "Clostridia"; Clostridiales; "Lachnospiraceae"; Roseburia,
Bacteria; "Firmicutes"; "Clostridia"; Clostridiales; "Lachnospiraceae"; nicht klassifizierte "Lachnospiraceae",
Bacteria; "Firmicutes"; "Clostridia"; Clostridiales; "Peptostreptococcaceae"; Sporacetigenium,
Bacteria; "Firmicutes"; "Clostridia"; Clostridiales; "Peptostreptococcaceae"; nicht klassifizierte "Peptostreptococcaceae",
Bacteria; "Firmicutes"; "Clostridia"; Clostridiales; "Ruminococcaceae"; Anaerofilum,
Bacteria; "Firmicutes"; "Clostridia"; Clostridiales; "Ruminococcaceae";
Anaerotruncus,
Bacteria; "Firmicutes"; "Clostridia"; Clostridiales; "Ruminococcaceae"; Oscillibacter,
Bacteria; "Firmicutes"; "Clostridia"; Clostridiales; "Ruminococcaceae"; nicht klassifizierte "Ruminococcaceae",
Bacteria; "Firmicutes"; "Clostridia"; Clostridiales; Clostridiaceae; "Clostridiaceae 1 "; Clostridium,
Bacteria; "Firmicutes"; "Clostridia"; Clostridiales; Clostridiaceae; "Clostridiaceae 1"; Sarcina,
Bacteria; "Firmicutes"; "Clostridia"; Clostridiales; Clostridiaceae; "Clostridiaceae 1"; nicht klassifizierte "Clostridiaceae 1",
Bacteria; "Firmicutes"; "Clostridia"; Clostridiales; Incertae Sedis XI; Anaerococcus,
Bacteria; "Firmicutes"; "Clostridia"; Clostridiales; Incertae Sedis XI; Finegoldia,
Bacteria; "Firmicutes"; "Clostridia"; Clostridiales; Incertae Sedis XI; Peptoniphilus,
Bacteria; "Firmicutes"; "Clostridia"; Clostridiales; Incertae Sedis XI; nicht klassifizierte Incertae Sedis XI,
Bacteria; "Firmicutes"; "Clostridia"; Clostridiales; Incertae Sedis XIII; Anaerovorax,
Bacteria; "Firmicutes"; "Clostridia"; Clostridiales; Incertae Sedis XIII; Mogibacterium,
Bacteria; "Firmicutes"; "Clostridia"; Clostridiales; Incertae Sedis XIV; Blautia,
Bacteria; "Firmicutes"; "Clostridia"; Clostridiales; nicht klassifizierte Clostridiales,
Bacteria; "Firmicutes"; "Clostridia"; Clostridiales; Veillonellaceae; Veillonella,
Bacteria; "Firmicutes"; "Erysipelotrichi"; "Erysipelotrichales"; Erysipelotrichaceae; Holdemania, und
Bacteria; "Proteobacteria"; Betaproteobacteria; Burkholderiales; Alcaligenaceae; Parasutterella.

5. Zusammensetzung gemäß irgendeinem der Ansprüche 1 bis 3, **dadurch gekennzeichnet, dass** der bakterielle Gehalt der Zusammensetzung eine oder mehrere der in Anspruch 4 genannten Bakteriengruppen einschließt.

6. Zusammensetzung gemäß irgendeinem der Ansprüche 1 bis 5 zur Verwendung bei der Behandlung von Diarrhö, wie beispielsweise Reizdarmsyndrom ("Irritable Bowel Syndrome", IBS) oder Antibiotikaassoziierte Diarrhö (AAD), insbesondere im Zusammenhang mit Clostridium difficile, Clostridium-difficile-assoziierte Diarrhö (CDAD) und rezidivierende Clostridium-difficile-Infektionen (RCDI).

7. Zusammensetzung gemäß irgendeinem der Ansprüche 1 bis 6 zur Verwendung bei der Verabreichung an einen Menschen durch eine Vorrichtung, ausgewählt aus nasoduodenaler Sonde, Gastroskop, Koloskop/Sigmoidoskop und Klistier, oder in gefriergetrockneter Form in einer geeigneten galenischen Präparation, z.B. Magensäure-resistenten Kapsel, Nano-Verkapselung oder Suppositorium.

8. Verfahren zur Herstellung einer Zusammensetzung, welche streng anaerob kultivierte humane intestinale Mikrobiota, ausgewählt aus wenigstens drei der vier Stämme (Phyla) Bacterioidetes, Firmicutes, Proteobacteria und Actinobacteria, umfasst, wobei das Verfahren **gekennzeichnet ist durch** die anaeorobe Kultivierung einer Stuhlprobe in einem Kulturmedium, welches die Proliferation der anaeoroben Bakterien von wenigstens drei der folgenden vier Stämme (Phyla) fördert: Bacterioidetes, Firmicutes, Proteobacteria und Actinobacteria, und **dadurch** gekennzeichnet ist, dass aerobe Bakterien während der Kultivierung unterdrückt werden.

## Revendications

1. Composition comprenant un microbiote intestinal humain cultivé strictement dans des conditions anaérobies sélectionné parmi au moins trois des quatre phylums Bacteroidetes, Firmicutes, Proteobactéria et Actinobacteria pour une utilisation dans la prévention et le traitement de maladies.

2. Composition selon la revendication 1, **caractérisée en ce qu'**elle est exempte de métallo-bêta-lactamases et de bêtalactamases à spectre élargi, de métabolites de substances pharmaceutiques ou de xénobiotiques.

3. Composition selon l'une quelconque des revendications 1 à 2, **caractérisée en ce qu'**elle est exempte des microbes suivants : virus de l'hépatite A, B et C, cytomégalovirus, virus d'Epstein-Barr, virus de l'immunodéficience humaine (VIH), Calici- et Rotavirus, Salmonella, Shigella, Campylobacter, Yersinia, et kystes de protozoaires.

4. Composition selon l'une quelconque des revendications 1 à 3, **caractérisée en ce que** les espèces bactériennes suivantes comprennent ensemble plus de 1 % de la teneur en bactéries totale de l'invention :
Bacteria;"Actinobacteria";Actinobacteria;Coriobacteridae;Co riobacteriales;"Co riobacterineae";Coriobacteriaceae;Collinsella,
Bacteria;"Actinobacteria";Actinobacteria;Coriobacteridae;Co riobacteriales;"Co riobacterineae";Coriobacteriaceae; Eggerthella,
Bacteria;"Actinobacteria";Actinobacteria;Coriobacteridae;Co riobacteriales;"Co riobacterineae";Coriobacteriaceae;Olsenella,
Bacteria;"Bacteroidetes";"Bacteroidia";"Bacteroidales";"Por phyromonadaceae
";Parabacteroides,
Bacteria;"Bacteroidetes";"Bacteroidia";"Bacteroidales";"Pre votellaceae";
Prevotella,
Bacteria;"Bacteroidetes";"Bacteroidia";"Bacteroidales";"Rik enellaceae";Alistipes,
Bacteria;"Bacteroidetes";"Bacteroidia";"Bacteroidales";Bact eroidaceae;
Bacteroides,
Bacteria;"Firmicutes";"Bacilli";"Lactobacillales";"Enteroco ccaceae";
Enterococcus,
Bacteria;"Firmicutes";"Clostridia";Clostridiales;"Eubacteri aceae";Eubacterium,
Bacteria;"Firmicutes";"Clostridia";Clostridiales;"Eubacteri aceae"; non classifiés _
"Eubacteriaceae",
Bacteria;"Firmicutes";"Clostridia";Clostridiales;"Lachnospi raceae";Anaerostipes,
Bacteria;"Firmicutes";"Clostridia";Clostridiales;"Lachnospi raceae";Dorea,
Bacteria;"Firmicutes";"Clostridia";Clostridiales;"Lachnospi raceae";Roseburia,
Bacteria;"Firmicutes";"Clostridia";Clostridiales;"Lachnospi raceae"; non classifiés
_"Lachnospiraceae",
Bacteria;"Firmicutes";"Clostridia";Clostridiales;"Peptostre ptococcaceae";Spor
acetigenium,
Bacteria;"Firmicutes";"Clostridia";Clostridiales;"Peptostre ptococcaceae";non classifiés_"Peptostreptococcaceae",
Bacteria;"Firmicutes";"Clostridia";Clostridiales;"Ruminococ caceae";Anaerofilum,
Bacteria;"Firmicutes";"Clostridia";Clostridiales;"Ruminococ caceae";
Anaerotruncus,
Bacteria;"Firmicutes";"Clostridia";Clostridiales;"Ruminococ caceae";Oscillibacter,
Bacteria;"Firmicutes";"Clostridia";Clostridiales;"Ruminococ caceae";non classifiés_"Ruminococcaceae",
Bacteria;"Firmicutes";"Clostridia";Clostridiales;Clostridia ceae;"Clostridiaceae1 ";Clostridium,
Bacteria;"Firmicutes";"Clostridia";Clostridiales;Clostridia ceae;"Clostridiaceae1
";Sarcina,
Bacteria;"Firmicutes";"Clostridia";Clostridiales;Clostridia ceae;"Clostridiaceae1 "; non classifiés_"Clostridiaceae1",
Bacteria;"Firmicutes";"Clostridia";Clostridiales;IncertaeSe disXI;Anaerococcus,
Bacteria;"Firmicutes";"Clostridia";Clostridiales;IncertaeSe disXI;Finegoldia,
Bacteria;"Firmicutes";"Clostridia";Clostridiales;IncertaeSe disXI;Peptoniphilus,
Bacteria;"Firmicutes";"Clostridia";Clostridiales;IncertaeSe disXI; non classifiés_In
certaeSedisXI,
Bacteria;"Firmicutes";"Clostridia";Clostridiales;IncertaeSe disXIII;Anaerovorax,
Bacteria;"Firmicutes";"Clostridia";Clostridiales;IncertaeSe disXIII;Mogibacterium,
Bacteria;"Firmicutes";"Clostridia";Clostridiales;IncertaeSe disXIV;Blautia,
Bacteria;"Firmicutes";"Clostridia";Clostridiales; non classifiés Clostridiales,
Bacteria;"Firmicutes";"Clostridia";Clostridiales;Veillonell aceae;Veillonella,
Bacteria;"Firmicutes";"Erysipelotrichi";"Erysipelotrichales ";Erysipelotrichaceae
;Holdemania, et
Bacteria;"Proteobacteria";Betaproteobacteria;Burkholderiale s;Alcaligenaceae;
Parasutterella.

5. Composition selon l'une quelconque des revendications 1 à 3, **caractérisée en ce que** le contenu bactérien de la composition comprend un ou plusieurs des groupes de bactéries mentionnés dans la revendication 4.

6. Composition selon l'une quelconque des revendications 1 à 5, pour une utilisation dans le traitement d'une diarrhée, telle que le syndrome du côlon irritable (SCI), ou une diarrhée associée aux antibiotiques (DAA), en particulier associée à Clostridium difficile, une diarrhée associée à Clostridium difficile (DACD) et les infections récurrentes à Clostridium difficile (IRCD).

7. Composition selon l'une quelconque des revendications 1 à 6, pour une utilisation dans l'administration à un être humain par un dispositif choisi parmi un tube nasoduodénal, un gastroscope, un colo/sigmoïdéoscope, et un lavement ou sous une forme lyophilisée dans une préparation galénique appropriée, par exemple une capsule résistant à l'acide gastrique, une nano-capsule ou un suppositoire.

8. Procédé de préparation d'une composition comprenant un microbiote intestinal humain cultivé strictement dans des conditions anaérobies sélectionné parmi au moins trois des quatre phylums Bacteroidetes, Firmicutes, Proteobactéria et Actinobacteria, le procédé étant **caractérisé par** une culture anaérobie d'un échantillon de selles dans un milieu de culture qui favorise la prolifération de bactéries anaérobies d'au moins trois des quatre phylums suivants : Bacteroidetes, Firmicutes, Proteobactéria et Actinobacteria, et **caractérisé en ce que** les bactéries anaérobies sont supprimées au cours de la culture.
